(19) Europäisches Patentamt
European Patent Office
Office européen des brevets

(11) Publication number: 0 522 624 A1

# (12) EUROPEAN PATENT APPLICATION

(21) Application number: 92201850.2

(22) Date of filing: 24.06.92

(51) Int. Cl.5: **A61K 7/027**, A61K 7/48

(30) Priority: 02.07.91 GB 9114255

(43) Date of publication of application:
13.01.93 Bulletin 93/02

(84) Designated Contracting States:
AT BE CH DE DK ES FR GB GR IT LI NL PT SE

(71) Applicant: UNILEVER N.V.
Weena 455
NL-3013 AL Rotterdam(NL)
(84) BE CH DE DK ES FR GR IT LI NL PT SE AT

(71) Applicant: UNILEVER PLC
Unilever House Blackfriars P.O. Box 68
London EC4P 4BO(GB)
(84) GB

(72) Inventor: Dunphy, Patrick Joseph
Unilever Research Colworth Lab., Colworth House
Sharnbrook, Bedford(GB)
Inventor: Dunnet, Paul
Unilever Research Colworth Lab., Colworth House
Sharnbrook, Bedford(GB)

(74) Representative: Elliott, Peter William et al
Unilever plc, Patent Division Colworth House
Sharnbrook
Bedford MK44 1LO(GB)

## (54) Cosmetic stick.

(57) A water in oil emulsion lipstick composition comprising oil, water, and a polymeric thickening/gelling agent for increasing the viscosity of the aqueous phase, the polymeric thickening/gelling agent comprising a thickening biopolymer, a thickening synthetic biopolymer, a heat setting biopolymer, a cool setting biopolymer, and a chemical setting biopolymer, or mixtures thereof.

EP 0 522 624 A1

## FIELD OF THE INVENTION

The invention relates to a cosmetic composition, particularly to a composition which is adapted to be applied to human skin. The composition is especially suitable for products for use on the lips, for depositing thereon a protective and/or therapeutic and/or coloured film . The invention is accordingly particularly concerned with an improved lipstick having an enhanced in-use feel, moisturisation, protection and reparative properties.

## BACKGROUND AND PRIOR ART

In conventional techniques used to manufacture make up products, for example lipstick products, fats and/or oils, together with pigments and/or lakes, and other non-aqueous ingredients, are usually added to a wax base which is melted to enable the ingredients to be thoroughly mixed and is then cast into moulds which, after cooling, provide the shaped moulded products.

In the manufacture of commercially available lipsticks, water is not usually incorporated into the lipstick formulation, and therefore, the lipstick when applied to the lips does not necessarily possess the smooth, soft attributes associated with other skin treatment products, such as skin creams, particularly those intended for moisturing the lips.

It is accordingly desirable to provide for some users a lipstick that has more of the moisturising attributes of skin products, such as those referred to above, than conventional water-free lipsticks.

It is proposed in GB 1,442,426 (Shiseido) that a cosmetic stick for use on human skin can be made, which comprises a water-in-oil emulsion comprising up to 50% water, 1 to 10% of polyhydroxyl gel such as glycerol, mannitol, dulcitol and carbohydrates, 1 to 5% of a non-ionic surfactant, and at least 20% of a cosmetic base material.

Whilst formulations of this type provide adequate cosmetic sticks, these still do not meet the requirements of the more discerning user, with respect to the glide, hardness, and overall feel of the stick in use, in particular when the stick is used as a lipstick. There is therefore still room for improvement to be made in the properties of these cosmetic sticks.

It is accordingly with the avoidance of these disadvantages, particularly with improving moisturisation of the lips, and delivery thereto of skin care active ingredients that this invention is concerned.

## SUMMARY OF THE INVENTION

We have discovered that by employing as a structurant a polymeric thickening gelling agent, optionally in combination with one or more surfactant compounds, that a superior water in oil cosmetic composition, for example a lipstick, can be obtained.

## DEFINITION OF THE INVENTION

Accordingly, the invention provides a water-in-oil emulsion lipstick composition comprising oil, water, and a polymeric thickening/gelling agent for increasing the viscosity of the aqueous phase of the composition, the polymeric thickening/gelling agent comprising a thickening biopolymer, a thickening synthetic biopolymer, a heat setting biopolymer, a cool setting biopolymer, and a chemical setting biopolymer, and mixtures thereof.

According to a particulary preferred aspect of the invention, the composition additionally comprises an emulsifier.

## DISCLOSURE OF THE INVENTION

The invention is concerned with an improved cosmetic composition, particularly a lipstick, which comprises a special structurant system that imparts to the composition consumer-perceivable improved in-use benefits.

The function of the special structurant system is not only to provide these in-use benefits, but also to aid the delivery to the lips of active ingredients, such as those described herein, which can function to provide moisturisation, emolliency and other improvement.

Optionally, the composition may additionally comprise one or more emulsifiers. The physical structure of compositions according to the invention is not exactly known, but is though to be a water-in-oil emulsion. This is especially so in systems which comprise one or more emulsifiers. However, in these embodiments,

as well as those which do not contain one or more optional emulsifiers the polymeric thickening/gelling agent in the composition is thought to be acting to provide a mechanically stabilized matrix in which emulsified water is retained.

## POLYMERIC THICKENING/GELLING AGENT

The invention employs a polymeric thickening and/or gelling agent as a structurant.

Types of suitable polymeric thickening gelling and/or agents are:

- thickening biopolymers, for example xanthan, starch, and chemically modified starches, guar, or locust bean gum, sodium alginate, and sodium caseinate, at sufficient levels to provide thickened gel structures.
- known synergistic mixtures of biopolymers, such as locust bean gum or guar gum with xanthan.
- thickening/gelling synthetic polymers, for example Carbopols.
- gelling or heat setting biopolymers, for example egg albumin.
- cool setting biopolymers, such as gelatin or agar.
- chemically setting biopolymers of defined melting point. In this context, it is known that the melting point of various chemically setting biopolymers can be adjusted to a desired level by incorporating into the chemically setting biopolymer the appropriate amount of chemical reagent, for example (in the case of carrageenin gum) the appropriate level of potassium or sodium ions.

By varying the melting point of the chemically setting biopolymer thus employed, the feel of the cosmetic composition may be altered, in particular the feel on application to the lips.

Examples of chemical setting biopolymers, and the chemical reagent whose concentration determines the melting point of the chemical setting biopolymer, include;

| Chemical setting biopolymer | Chemical Reagent |
|---|---|
| kappa-carrageenin | potassium or sodium ions. |
| iota-carrageenin | potassium or calcium ion |
| alginate | calcium ion |
| pectin | sugar |
| low methoxy pectin | calcium ion |

Additionally, mixed biopolymer compositions based on phase separated systems may be used. These may include for example mixtures of enzymatically modified starch (e.g. PASELLI (trade mark)), with gelatin.

One or more of these polymeric thickening and/or gelling agents may be employed as the structurant. The total amount of polymeric thickening gelling agent in the system may comprise 0.1-10%, preferably 0.2-3%, of the total cosmetic composition.

## THE EMULSIFIER SYSTEM

The composition may also optionally comprise an emulsifier system, which may comprise one or more different emulsifiers, which may contribute to the appearance and stability of the composition.

Any type of emulsifier may be used, although preferred emulsifiers are phospholipids, glyceride derivatives, or mixtures thereof.

Examples of three classes of phospholipids are phosphoglycerides, lysophosphoglycerides and the important but smaller group of sphingomyelins. It is also possible to employ a mixture of two or more of these phospholipids, which may together then comprise the emulsifier system.

Examples of phosphoglycerides include:

- phosphatidyl choline,
- phosphatidyl ethanolamine,
- phosphatidyl serine,
- phosphatidyl inositol,
- disphosphatidyl glycerol,
- ammonium phosphatides (e.g. chocothin YN (Trademark) of general structure

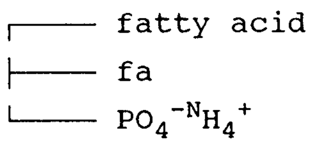

and

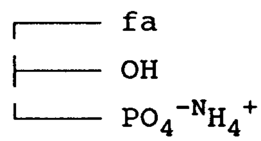

- lysophospholipids and phospholipids from egg containing more saturated fatty acids (e.g. Ovothin 160 (Trademark)) or synthetic analogues with saturated fatty acids from $C_{10}$ to $C_{20}$.
- mono and/or dialkyl and mono and di-alkenyl phosphates or mixtures of the same e.g. dicetyl and dioleyl phosphates and oleth-n-phosphates (where n = 3 to 50).
- sphingomyelins consisting of single fatty acyl chain conjugated via an amide linkage to the nitrogen of sphingosine which is again linked to the phosphoryl group of choline or other bases.
- ceramides in which the phosphoryl base head group of sphingomyelins is absent.
- cerebrosides and gangliosides which contain polysaccharide head groups in place of the phosphoryl base group of sphingomyelins.
- plasmogens in which the 1' position is occupied by an ether linked alk-1-enyl chain.
- cardiolipin in which two molecules of diacyl glycerol phosphatide are linked by a molecule of glycerol and mixtures thereof.

The most preferred phosphoglyceride is that known as lecithin, particularly soybean lecithin, which comprises a mixture of some of the above examples of specific phosphoglycerides.

Examples of lysophosphoglycerides includes:

lysophosphatidyl choline,
lysophosphatidyl ethanolamine,
lysophosphatidyl serine,
lysophosphatidyl inositol, and mixtures thereof

Another preferred type of emulsifier which may be used is a glyceride derivative. It is also possible to employ a mixture of two or more glyceride derivate emulsifiers, optionally in combination with other emulsifiers such as phospholipids.

Preferred glyceryl derivatives include:

i) monoacyl (mainly 1-substituted) glycerol, such as glyceryl monoalkanoates, in which the alkanoate group has from 2 to 20 carbon atoms, with or without one or more hydroxyl groups, and is branched or unbranched, specific examples of which are:

glyceryl caprylate
glyceryl caprate
glyceryl laurate
glyceryl myristate
glyceryl palmitate
glyceryl stearate
glyceryl isostearate

or glyceryl monoalkenoates, in which the alkenoate group has from 3 to 20 carbon atoms, with or without one or more hydroxyl groups, and is branched or unbranched, and has from 1 to 4 double bonds, specific examples of which are:

glyceryl oleate
glyceryl linoleate
glyceryl ricinoleate.

ii) diacyl (1,2- or 1,3- di substituted) glycerols, such as glyceryl dialkanoates, in which the alkanoate groups each have from 2 to 20 carbon atoms, with or without one or more hydroxyl groups, and each are branched or unbranched, specific examples of which are:

glyceryl diacetate
glyceryl dibutanoate
glyceryl dilaurate,

or glyceryl dialkenoates, in which the alkenoate groups each have from 3 to 20 carbon atoms with 1 to 4 double bonds, with or without one or more hydroxyl groups, and either branched or unbranched; specific examples include:

glyceryl dioleate
glyceryl dilinoleate.

iii) polyglyceryl esters with two to five linearly linked glyceryl units, to which are attached from 1 to 2

acyl groups each having from 2 to 20 carbon atoms, with or without one or more hydroxyl groups, either branched or unbranched; specific examples include polyglyceryl ricinoleate and diglycerol-1,6-dioleate.

Other preferred glycerol derivatives include:

- glycerol ether derivatives of the above mono and diacyl types, for example $CH_2OH.CHOH.CH_2OX$. Examples include where $X = CH_3(CH_2)_{17}$ (batyl alcohol), and where $X = CH_3(CH_2)_{15}CH = CH$ (selachyl alcohol).
- mixed acyl, ether derived glycerols, the neutral plasmogens of which are 1'-alk-1-enyl, 2'3' diacyl glycerols.
- polyhydric ethers and esters with saturated and unsaturated alkyl/alkenyl and acyl/acetyl chains and, mono, di and other derivates of ethylene, propylene, butylene and polyethylene glycols.
- similar derivatives to the above of polyhydric alcohols such as sorbitol, glucose, mannitol and sucrose.
- anionic, cationic and amphoteric surfactants.

Particularly preferred examples of the glyceride derivative emulsifiers are:

glyceryl caprate
glyceryl laurate
glyceryl oleate
glyceryl linoleate
glyceryl isostearate
glyceryl dilinoleate
glyceryl dicaprate

A glyceride derivative emulsifier can comprise one or more pure glycerides, but it is usually more convenient to employ glycerides derived from natural oils such as vegetable or seed oils, examples of which are sunflower seed oil, soybean oil, palm kernel oil and palm oil. Particularly preferred are monoglycerides of sunflower seed oil and of palm oil, and mono- and di-glycerides of soybean oil.

Further examples of the emulsifier include esters of fatty alcohols with a hydroxy acid, such as glyceryl citrate, cetyl citrate, cetyl lactate, and cetyl ricinoleate.

The total amount of emulsifier in the composition may range from 0 to 35%, preferably 0.2 to 15%, more preferably 0.2 to 10%, most preferably 0.5 to 5% of the total composition.

If the emulsifier comprises a phospholipid, the amount of phospholipid in the composition preferably comprises 0.1-10 % by weight of the composition, most preferably 0.2-5 % be weight.

If the emulsifier comprises a glyceride derivative, the amount of glyceride derivative in the composition preferably comprises 0.2-10 % by weight of the composition, most preferably 0.2-5 % by weight.

The presence of one or more emulsifiers in the composition in addition to the polymeric thickening gelling agent has been found to enhance the stability and performance of the cosmetic composition.

## The oil

The cosmetic composition of the invention also comprises an oily ingredient, hereinafter referred to as an "oil".

A chosen oil will normally be liquid at room temperature (i.e. 20°C), and can comprise a single oil or a mixture of two or more oils. Example of suitable oils include:

caprylic triglycerides
capric triglycerides
isostearic triglycerides
adipic triglycerides
propylene glycol myristyl ether acetate
lanolin oil
polybutene
isopropyl palmitate
isopropyl myristate
diethyl sebacate
diisopropyl adipate
hexadecyl stearate
cetyl oleate
oleyl alcohol
hexadecyl alcohol
wheatgerm oil
hydrogenated vegetable oils

petrolatum
modified lanolins
branched-chain hydrocarbons, alcohols and esters
castor oil
corn oil
cotton seed oil
olive oil
palm kernel oil
rapeseed oil
safflower seed oil
jojoba oil
evening primrose oil
avocado oil
mineral oil
volatile and non-volatile silicone oils
The amount of the oil normally present in the cosmetic composition of the invention is from 2 to 97%, preferably 30 to 90% by weight of the cosmetic composition emulsion.

**Crystalline Lipid Material**

Preferably, the composition comprises at least one crystalline lipid material. Preferred classes of crystalline lipid material are waxes, triglycerides and hydrocarbons, in particular waxes.

**Wax**

Examples of waxes include:
candelilla wax
ozokerite wax
carnauba wax
beeswax
spermaceti
cetyl alcohol
stearyl alcohol
lanolin.

The amount of crystalline lipid material when present in the cosmetic composition of the invention is up to 30%, usually from 1 to 25% and, preferably from 5 to 20% by weight of the composition.

Conveniently, when the crystalline lipid material is a wax, the wax has a melting point in the region 50-80 degrees centigrade, more preferably 55-75 degrees centigrade.

**Water and other water soluble components**

The cosmetic composition of the invention also comprises water. The water provides a solvent for any water-soluble ingredients present in the cosmetic composition.

The amount of water present in the cosmetic composition of the invention is from 1 to 95%, preferrably 2 to 50%, most preferably 2 to 35%. However, structuring systems according to the invention have been found to be especially suitable for use in lipstick compositions which contain a level of water over 10% of the composition

Additionally, it has been found that the properties of the aqueous phase may be beneficially modified by the addition of polyhydric alcohols, such as glycerol or sorbitol, at levels of around 10 to 75% by weight of the aqueous phase, and/or lamellae forming monoglycerides, based on C10 to C20 fatty acids, which may be saturated or unsaturated, branched or unbranched, but preferably C16 and C18 (palmitic and stearic acids).

**Skin care active ingredients**

The cosmetic emulsion of the invention can optionally also comprise skin care active ingredients which can improve the quality of skin, particularly dry or damaged skin especially the lips, or which can possess a therapeutic or pharmaceutical benefit. By way of example, the following skin care active ingredients can be

employed, in the amounts stated, in lip treatment compositions of the invention, especially in lipsticks.

| | % w/w of the composition |
|---|---|
| Zinc oxide | 1 to 2 |
| $\beta$-glycyrrhetic acid | 0.1 to 1 |
| camomile oil | 0.1 to 1 |
| ginko biloba extract | 0.1 to 1 |
| pyroglutamic acid, salts or esters | 0.5 to 5 |
| sodium hyaluronate | 0.1 to 5 |

The amount of skin care active ingredients when present in the cosmetic composition of the invention will normally be from 0.0001 to 10% by weight of the cosmetic composition. In general terms it can be stated that the amount of any skin care active ingredient can be that which is conventionally employed in products intended for topical application to human skin.

**Other ingredients**

The cosmetic composition of the invention can optionally also comprise other ingredients as conventionally employed in lipsticks, or other skincare products.

Examples of other ingredients include perfumes, antioxidants, colourants such as staining dyes and pigments, humectants, germicides, sunscreens, lipid materials and vitamins.

Particularly preferred pigments, when present, include calcium, barium and aluminium lakes, iron oxides, titanium dioxide, and mica.

Particularly preferred humectants include glycerol, sorbitol and other polyols.

Particularly preferred germicides include Triclosan.

Particularly preferred sunscreens include octyl methoxycinnamate and butyl methoxydibenzoylmethane.

Particularly preferred lipid materials include ceramides and liposomes.

Particularly preferred vitamins include vitamins A, C, D & E, and retinoic acid.

**Product form**

The composition according to the invention takes the form of a stick, which can be applied to the lips with a suitable applicator.

For example, when the cosmetic composition is a stick, the aqueous phase will usually form from 2 to 50%, preferably from 5 to 15% by volume and the oily phase including wax ingredients, will form from 98 to 50, preferably 95 to 85% by volume of the composition.

A particularly preferred embodiment of the invention in a water-in-oil emulsion lipstick comprising:

i from 30 to 97% by weight of oil
ii from 1 to 25% by weight of wax
iii from 1 to 20% by weight of water
iv from 0.2 to 3% of a polymeric thickening/gelling agent.
v from 0.2 to 5% of an emulsifier system.
vi a sufficient amount of pigment.

**Use of the composition**

The lipstick composition of the invention is ideally suited for use in treating the lips, especially for applying to the lips a permanent or semi-permanent colour ideally with a gloss or lustre finish. The composition can also be used in treating the lips with a skin care agent for protection against exposure to adverse weather, including the wind and the rain or exposure to excessive doses of sunlight .

The lipstick composition can accordingly be applied to the lips in the traditional manner using a convenient holder or applicator to provide a decorative and/or protective film thereto.

**Examples**

Lipstick formulations were prepared according to the following method, for formulations detailed below.

1. The combined wax, oil and the optional emulsifier, which comprise the oily phase (A), were heated

and stirred in a sealed vessel to a temperature of 90 °C, until the mass had melted.

2. The melt so obtained was then cooled to 75-80 °C and stirred under vacuum to remove air.

3. The product aqueous phase, comprising the water, and other water soluble ingredients, and the polymeric thickening and/or gelling agent (B), were heated gently (for example to around 75-80 °C) in such a way as to develop the functionality of the polymeric thickening and/or gelling agent. The mixture was then added slowly, with continued mixing, to the molten oily phase (A), and the mixture homogenised for 5 to 10 minutes.

4. Pigments and fragrance dispersed in castor oil (C) were finally added and the water-in-oil emulsion so formed was further deaerated prior to cooling.

5. The deaerated emulsion was finally poured into moulds and cooled to form lipsticks.

Example 1

Lipstick formulations with cool setting biopolymer (gelatin)

| | parts by weight of the composition |
|---|---|
| **Wax** | |
| Candelilla Wax | 6.4 |
| Ozokerite Wax | 3.2 |
| Beeswax | 4.0 |
| Carnauba Wax | 0.4 |
| Lanolin | 4.8 |
| **Oil** | |
| Caprylic/Capric triglyceride | 6.0 |
| Propylene glycol myristyl ether acetate | 6.0 |
| Lanolin Oil | 2.4 |
| Polybutene | 0.8 |
| Softisan 649 (*) | 9.2 |
| Isopropyl palmitate | 12.0 |
| **Emulsifier System** | |
| Phospholipid (Soya Lecithin) | 1.0 |
| Sunflower oil monoglyceride | 4.0 |
| **Aqueous phase** | |
| Gelatin (270 bloom strength) | 0.36 |
| Water | 9.64 |

* - Softisan 649 is a mixture of esters of natural fatty acids of isostearic acids and adipic acids, ex. Hulse

| Pigments distributed in castor oil | |
|---|---|
| Titanium dioxide | 5.0 |
| Colourants | 3.4 |
| Castor oil | 21.4 |

Example 2

Lipstick formulation with chemical setting biopolymer

This formulation is the same as example 1, except that the aqueous phase comprises;

| composition | parts by weight of the |
|---|---|
| Water<br>0.1 M Potassium chloride<br>carrageenan | 8.88<br>0.97<br>0.15 |

This lipstick has a melting point of approximately 35° C. However the exact melting point of the lipstick can be varied by varying the amount of potassium chloride in the aqueous phase, and can readily be made to be for example from 25° C up to around 65° C. In the formulation iota or kappa carrageenan can readily be substituted for carrageenan to provide lipstick formulations with desired melting points.

Example 3

Lipstick formulation with thickening biopolymer

This formulation is the same as example 1, except that the aqueous phase comprises;

| composition | parts by weight of the |
|---|---|
| Water<br>Xanthan | 9.9<br>0.1 |

## Claims

1. A water in oil emulsion lipstick composition comprising oil, water, and a polymeric thickening/gelling agent for increasing the viscosity of the aqueous phase, the polymeric thickening/gelling agent comprising a thickening biopolymer, a thickening synthetic biopolymer, a heat setting biopolymer, a cool setting biopolymer, and a chemical setting biopolymer, or mixtures thereof.

2. A lipstick composition according to claim 1, wherein the thickening/gelling agent comprises 0.1-10% by weight of the compositon.

3. A lipstick composition according to claim 1 or claim 2, wherein the thickening/gelling agent is a thickening biopolymer comprising xanthan gum, starch, a chemically modified stach,guar gum, locust bean gum, sodium alginate, sodium caseinate, or mixtures thereof.

4. A lipstick composition according to claim 1 or claim 2, wherein the thickening/gelling agent is a thickening synthetic biopolymer comprising a carbopol.

5. A lipstick compositon according to claim 1 or claim 2, wherein the thickening/gelling agent is a heat setting biopolymer comprising egg ealbumin.

6. A lipstick composition according to claim 1 or claim 2, wherein the thickening/gelling agent is a cool setting biopolymer comprising gelatin or agar.

7. A lipstick composition according to claim 1 or claim 2, wherein the thickening/gelling agent is a chemical setting biopolymer comprising kappa carrageenan, iota carrageenan, alginate, pectin, or low methoxy pectin.

8. A composition according to any of the preceding claims, additionally comprising up to 35% by weight of an emulsifier.

9. A lipstick composition according to claim 8, wherein the emulsifier comprises a phospholipid, a glyceride derivative, or mixtures thereof.

10. A lipstick composition according to claim 9, wherein the phospholipid emulsifier is lecithin.

11. A lipstick composition according to claim 9, wherein the glyceride derivative emulsifier isglyceryl caprate, glyceryl laurate, glyceryl oleate, glyceryl linoleate, glyceryl isostearate, glyceryl dilinoleate, or glyceryl dicaprate.

12. A lipstick composition according to claim 9, wherein the glyceryl derivative is a monoglyceride of sunflower seed oil or palm oil, or mono- or diglycerides of soybean oil.

13. A lipstick composition according to any of the preceding claims, wherein the composition additionally contains up to 30 % of a wax.

14. A lipstick compositon according to any of the preceding claims, wherein the copositon comprises from 2-30% of water.

15. Use of a thickening/gelling agent comprising a thickening biopolymer, a thickening synthetic biopolymer, a heat setting biopolymer, a cool setting biopolymer, or a chemical setting biopolymer as a structuring agent in a water-in-oil emulsion lip stick composition.

European Patent Office

# EUROPEAN SEARCH REPORT

Application Number

EP 92 20 1850

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | US-A-3 957 969 (Y. FUJIYAMA ET AL.)<br>* column 1, line 1 - line 68 *<br>* column 2, line 1 - line 47 *<br>* claims 1-14 *<br>--- | 1,2,3,8, 9,11, 13-15 | A61K7/027<br>A61K7/48 |
| X | EP-A-0 373 838 (ICI AMERICAS INC.)<br>* page 2, line 5 - line 10 *<br>* page 3, line 32 - line 35 *<br>* page 7 *<br>--- | 1,2,4,8, 15 | |
| Y | FR-A-2 466 273 (J. GONZALES)<br>* page 1, line 1 - line 10 *<br>* page 4, line 4 - line 40 *<br>* examples 1,3,4 *<br>* claims 1-6 *<br>--- | 1-3, 7-10,14, 15 | |
| Y | FR-A-977 547 (J. DE GRANVILLE)<br>* the whole document *<br>----- | 1-3, 7-10,14, 15 | TECHNICAL FIELDS SEARCHED (Int. Cl.5 )<br>A61K |

The present search report has been drawn up for all claims

| Place of search | Date of completion of the search | Examiner |
|---|---|---|
| THE HAGUE | 26 OCTOBER 1992 | SIERRA GONZALEZ |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)